(19) Europäisches Patentamt
European Patent Office
Office européen des brevets

(11) EP 2 037 800 B1

(12) EUROPEAN PATENT SPECIFICATION

(45) Date of publication and mention
of the grant of the patent:
**02.06.2010 Bulletin 2010/22**

(51) Int Cl.:
***A61B 5/0205*** (2006.01)

(21) Application number: **07747542.4**

(22) Date of filing: **29.06.2007**

(86) International application number:
**PCT/NL2007/050320**

(87) International publication number:
**WO 2008/002141 (03.01.2008 Gazette 2008/01)**

(54) **METHOD AND ANALYTICAL DEVICE FOR THE ANALYSIS OF RESPIRATION**

VERFAHREN UND ANALYSEVORRICHTUNG ZUR ANALYSE DER ATMUNG

PROCEDE ET DISPOSITIF D'ANALYSE DESTINES A L'ANALYSE DE LA RESPIRATION

(84) Designated Contracting States:
**AT BE BG CH CY CZ DE DK EE ES FI FR GB GR
HU IE IS IT LI LT LU LV MC MT NL PL PT RO SE
SI SK TR**

(30) Priority: **30.06.2006 NL 2000118**

(43) Date of publication of application:
**25.03.2009 Bulletin 2009/13**

(73) Proprietor: **R&S Techmedic B.V.
1721 PC, Broek Op Langedijk (NL)**

(72) Inventors:
• **BREST VAN KEMPEN, Rutger, Alexander
NL-1723 HR Noord Scharwoude (NL)**
• **HUNTELERSLAG, Alexander, Adriaan
NL-1722 KE Zuid-scharwoude (NL)**

(74) Representative: **Ketelaars, Maarten F.J.M. et al
Nederlandsch Octrooibureau
J.W. Frisolaan 13
2517 JS Den Haag (NL)**

(56) References cited:
**DE-A1- 19 718 806      US-A- 5 810 722
US-A1- 2005 119 711**

• **DE CHAZAL P ET AL: "Automatic sleep apnoea
detection using measures of amplitude and heart
rate variability from the electrocardiogram"
PATTERN RECOGNITION, 2002. PROCEEDINGS.
16TH INTERNATIONAL CONFERENCE ON
QUEBEC CITY, QUE., CANADA 11-15 AUG. 2002,
LOS ALAMITOS, CA, USA,IEEE COMPUT. SOC,
US, vol. 1, 11 August 2002 (2002-08-11), pages
775-778, XP010613446 ISBN: 0-7695-1695-X**

## Description

FIELD OF THE INVENTION

The present invention relates to a method and an analytical device for the analysis of respiration.

PRIOR ART

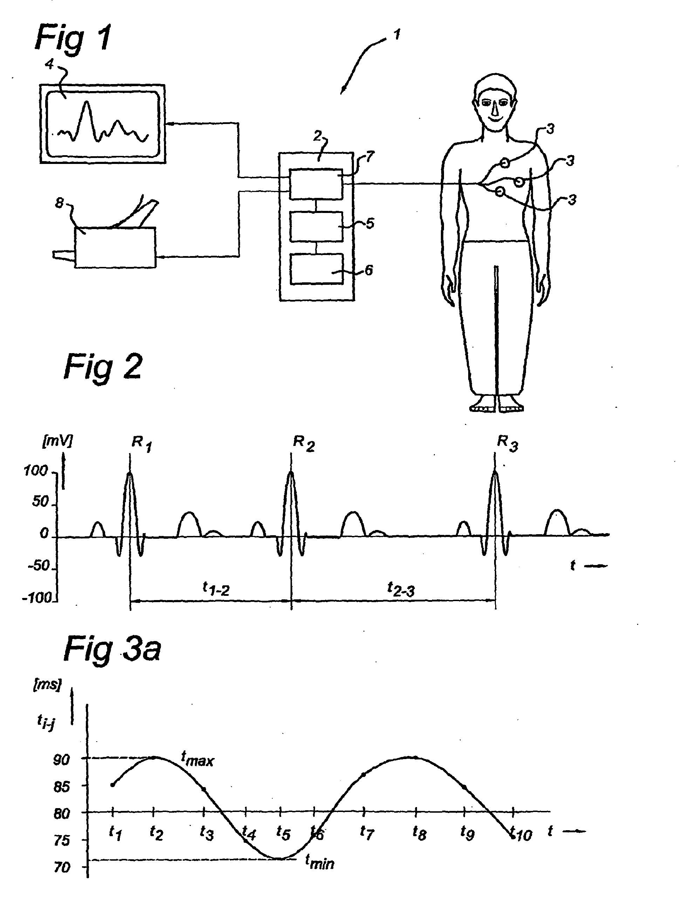

Electrocardiographs (ECG machines) are known from the prior art. With such ECG machines, information can be obtained about the heart of, for example, a human being or an animal, and in particular about the functioning of their heart. This information is obtained by measuring the electrical signals of the heart with the aid of electrodes attached to the skin. This information can indicate, amongst other things, irregularities of the heart (such as an abnormal, a slow or a fast heartbeat). The operation of an ECG machine according to the prior art will be described later in more detail with reference to Fig. 1.

The aim is to provide a method and an analytical device with which information can be obtained about the respiration of a person or an animal.

From US 2005/0119711 is known a device for monitoring sleep disordered breathing by monitoring heart rate.

BRIEF DESCRIPTION

According to one aspect, an analytical device is provided for the analysis of respiration, where the analytical device comprises a processor and an input-output unit, and the analytical device is arranged for

- the reception, via an input-output unit, of measuring data that are representative of the electrical activity of the heart,
- the determination, from the measuring data, of a number of periods of time between successive heartbeats. It has been found that the number of periods of time found to elapse between successive heartbeats can be very useful for obtaining information about a person or animal, in particular information about the respiration of the said person or animal.

The analytical device can receive measuring data from a number of electrodes that can be placed on the body. This is a simple way of obtaining information about the electrical activity of the heart.

The analytical device can receive measuring data from one of the following: an ECG machine (1), a floppy disk, CD-ROM, DVD or USB stick, or another suitable medium.

The analytical device can receive the measuring data for example from an ECG machine in various other ways. The analytical device can also receive data from a data carrier on which measuring data about the heart were stored in a previous stage.

The analytical device is arranged for receiving measuring data at a sampling rate of 1 ms or less, e.g. 0.1 ms. In order to obtain accurate information about the respiration of a person or animal, the measuring data that are representative of the electrical activity of the heart are preferably collected at the highest possible sampling rate, so that the periods of time between successive heartbeats can be determined with sufficient accuracy.

The analytical device is also set up out putting or the measuring data that are representative of the electrical activity of the heart to, for example, an ECG machine. This makes it possible to provide an analytical device that can function in cooperation with an ECG machine.

The analytical device may also be arranged for

- the determination of the respiratory rate from the determined number of periods of time between successive heartbeats. This can be done by finding regular variations in the found to elapse times between successive heartbeats. When this time increases, one is generally faced with inhalation, and when this time decreases, one is generally faced with exhalation. By detecting inhalation and exhalation consecutively, the respiratory rate, for example the number of breaths per minute, can be determined.

According to a further aspect, the analytical device is arranged for

- the determination of a maximum value of the time between successive heartbeats from the measuring data,
- the determination of a minimum value of the time between successive heartbeats from the measuring data, and
- the calculation of a measure (B) of the respiratory effort, where the measure (B) is proportional to the difference between the maximum value and the minimum value: $B = t_{max} - t_{min}$. When determining a maximum and minimum value, interpolation techniques can be used. The measure can provide valuable information about the state of a human being or an animal.

The analytical device is further arranged for

- the reading-in of a reference measure and
- the calculation of an indicator that shows whether the someone has a respiratory effort much above or that is below the average, where the indicator is calculated from the reference measure and the measure. This can be done in various ways, for example by using $I = B - B_{ref}$ or $I = B - B_{ref}/B_{ref}$. Naturally, other calculations are also possible. Such an indicator I provides a quick and readily understandable indication of the effort that a person or animal must exert in order to breathe.

The analytical device may also be arranged for outputting, via the input-output unit, at least one of a number of periods of time between successive heartbeats, a respiratory rate, a measure and an indicator to a suitable output medium such as a screen or a printer. In this way, the data collected by the analytical device can be made visible in a simple manner.

The analytical device may be integrated with an electrocardiogram machine. The analytical device can be integrated with an electrocardiogram machine in a simple way. This provides a compact system that can give both information about the functioning of the heart and information about respiration.

According to one aspect, a method is provided, which comprises:

- the reception of measuring data that are representative of the electrical activity of the heart,
- the determination of a number of periods of time between successive heartbeats from the measuring data.

The measuring data may be received from a number of electrodes placed on the body.

The measuring data may be received from one of the following: an ECG machine, a floppy disk, CD-ROM, DVD or USB stick, or another suitable medium.

The measuring data received can have a sampling rate of 1 ms or less, e.g. 0.1 ms.

The method further may comprise:

- the determination of the respiratory rate from the number of periods of time found to elapse between successive heartbeats.

The method also comprises:

- the determination of a maximum value of the period of time between successive heartbeats from the measuring data,
- the determination of a minimum value of the period of time between successive heartbeats from the measuring data, and
- the calculation of a measure (B) of the respiratory effort, where the measure (B) is proportional to the difference between the maximum and the minimum value: $B = t_{max} - 1_{min}$.

The method also comprises:

- the reading-in of a reference measure and
- the calculation of an indicator that shows whether someone has a respiratory effort that is much above or below the average, where the indicator is calculated from the reference measure and the measure.

The method may also comprise outputting one of a number of periods of time between successive heartbeats, a respiratory rate, measure and indicator.

According to one aspect, a computer program is provided that, when loaded onto an analytical device, enables the processor of the analytical device to carry out one of the methods as claimed.

According to one aspect, a data carrier is provided that contains a computer program as claimed.

BRIEF DESCRIPTION OF THE DRAWINGS

The invention will be explained below in more detail with the aid of drawings that illustrate some exemplary embodiments. The drawings are only intended for purposes of illustration and not for limiting the scope of protection, which is defined in the claims.

Figure 1 shows a device according to the prior art,
Figure 2 shows an ECG record,
Figures 3a and 3b show graphs according to one embodiment,
Figure 4 shows a device according to one embodiments,
Figures 5-7 show flow charts according to different embodiments,
Figure 8 shows an ECG record.

DETAILED DESCRIPTION

Figure 1 schematically shows an ECG machine 1 according to the prior art. Such an ECG machine 1 can produce an electrocardiogram ("heart film"). Such an ECG machine 1 generally comprises a processing unit 2 and a number of electrodes 3, for example three, which are connected to the processing unit 2 and can be attached to the skin of a human being or an animal. The electrodes 3 register the electrical activity (electrical voltage of the order of 1 millivolt) of the heart as a function of time and pass it on to the processing unit 2. The processing unit 2 can be set up, for example, for generating an electrocardiogram and for example for displaying a graph on a screen 4.

The processing unit 2 can be a computer that comprises a processor 5, a memory 6 and an input-output unit 7, where the memory 6 can comprise, amongst other things, program instructions that can be read and carried out by the processor 5 and which enable the processor 5 to receive data from the electrodes 3 via the input-output unit 7 and to convert these into data that can be output, via the input-output unit 7, to a suitable output medium, such as a screen 4 or a printer 8. The processing unit 2 can process the signals from the electrodes 3, for example by sampling, filtering or amplifying them at a sampling rate of 20 ms (= 20 milliseconds). The (real-time) signals coming from the various electrodes 3 can also be processed separately.

Using such an ECG machine 1 it is possible to obtain information about the functioning of the heart, such as the number of heartbeats per minute, irregularities in the cardiac rhythm, etc.

Fig. 2 schematically shows an example of an electrocardiogram of the type that can be displayed by a screen 4. The expert will be able to distinguish the various cycles of the heartbeat in the electrocardiogram, as shown. The cardiac cycle is known to comprise two phases - the systole and the diastole. During the systole, the chambers of the heart contract whereby blood is pumped into the aorta. In the diastole, the cardiac muscle relaxes, and the chambers of the heart are filled up again.

Clearly distinguishable peaks can be seen in the electrocardiogram shown in Fig. 2. These peaks are called R peaks, and it is known that the mechanical systole begins a very short time after such an R peak. The successive R peaks are denoted as $R_1$, $R_2$, $R_3$, etc.

Since each heartbeat generally comprises one R peak, it is possible to use the recording of the R peaks in the electrocardiogram for determining the number of heartbeats per minute, and irregularities in this frequency can also be detected.

It is known that the normal human heart beats about 70-80 times a minute. It is also known that a human being breathes about 15 times a minute. These normal values can differ widely in the case of diseases. According to the embodiments discussed below, a device is provided with which information about breathing can also be obtained, especially the number of breaths a minute, in the case of a human being or an animal. Use is made here of the realization that successive values of the RR periods (or periods of time) $t_{i-j}$ are not constant but vary with, amongst other things, the respiration.

Embodiment 1

According to one embodiment, the periods between successive R peaks are measured. The time elapsing between a first R peak $R_i$ at time $t_i$ and a second R peak $R_j$ at time $t_j$ is denoted as the RR period (or period of time) $t_{i-j}$. Therefore, the RR period (period of time) $t_{1-2}$ is the time elapsing between an R peak $R_1$ and the next R peak $R_2$.

This embodiment makes use of the realization that the successive values of the RR periods $t_{i-j}$ are not constant but vary with, amongst other things, the respiration. The value of the RR period $t_{i-j}$ varies in time about a mean value, and the variation is connected with the respiration, amongst other things. If the successively measured values of the RR period $t_{i-j}$ are plotted against time $t_j$, a picture as shown schematically in Fig. 3a is obtained.

Fig. 3a shows a graph in which the successively measured values of the RR period $t_{i-j}$ are plotted along the vertical axis against time $t_j$. This gives a sinusoidal curve. As can be seen in Fig. 3a, each period of the sinusoidal curve comprises a number of measuring values. The sinusoidal curve can be obtained by means of interpolation. It will also be realized that a measuring point will not exactly coincide with each crest and each trough.

However, this can be compensated by taking the mean of the sinusoidal curve over a number of breaths. Assuming that breathing is relatively constant during this time, the crests and troughs without an exact measuring point can be determined by making use of measurements from previous breaths.

As has already be mentioned above, the curve shown in Fig. 3a by way of example can be obtained by means of interpolation, and the crests and troughs need not necessarily coincide here with the actual measuring values of the RR periods $t_{i-j}$.

More than one period of the curve shown in Fig. 3a can be reckoned with here. The curve can be determined relatively accurately by applying interpolation to the measuring values $t_{i-j}$ over a relatively long period.

The variation in the RR period $t_i$.j is connected with the breathing of human beings or animals. The values of the RR periods $t_{i-j}$ show directly in which cycle of the respiration the impulses measured by the electrodes 3 lie, and so the respiratory cycle of the patient can be identified: the RR period $t_{i-j}$ decreases on inhalation and the RR period $t_{i-j}$ increases on exhalation. The descending parts of the curve in Fig. 3a then correspond to inhalation, and the ascending parts of the curve in Fig. 3a correspond to exhalation.

However, it should be realized that, in certain cases and in certain disorders, the relationship between the respiratory cycle and the variation in the RR period $t_{i-j}$ can be different, for example it can be the opposite of that mentioned in the previous paragraph, with the RR period $t_{i-j}$ decreasing on exhalation, and the RR period $t_{i-j}$ increasing on inhalation. The ascending parts of the curve in Fig. 3a then correspond to inhalation, and the descending parts of the curve in Fig. 3a correspond to exhalation.

With a cardiac frequency of 60 heartbeats per minute, the values of the RR periods are of the order of one second, but the variation in the RR period $t_{i-j}$ is of the order of milliseconds, for example 3, 5 or 10 ms. To derive the variations of the RR period $t_{i-j}$ from an electrocardiogram, a sufficiently high sampling rate of the analytical device 1 is chosen. ECG machines 1 with a sampling rate of 20 ms are known from the prior art. The analytical device according to the embodiments discussed here can use a relatively high sampling rate, for example a sampling rate of 1 or 0.1 ms or even less is chosen. Such a high sampling rate makes it possible to derive the variations in the RR period $t_{i-j}$ from the measuring data obtained from the electrodes 3 and so derive information about breathing, such as for example the respiratory rate, shortness of breath (dyspnoea), respiratory effort, respiratory work, fitness, and other parameters related to lung function.

As a result of this high sampling rate, the time between 2 successive heartbeats can be determined with sufficient accuracy.

In the embodiment described here, the various heartbeats are detected by identifying the R peaks. However, it will be realized that the heartbeats can also be detected at other points in the ECG generated by the electrodes 3.

This embodiment can take various forms. For example, the analytical device can be integrated with the ECG machine 1 as discussed with reference to Fig. 1. Such an ECG machine 1 can be made adapted for performing the embodiments described here by storing suitable program instructions in its memory 6, where the instructions can be read and implemented by the processor 5 and they enable the processor 5 both to receive data from the electrodes 3 via the input-output unit 7 and to convert these into data about breathing, such as the respiratory rate and other derived parameters. These data can be output, via the input-output unit 7, to a suitable output medium, such as a screen 4 or a printer 8. The data output can be for example in the form of an image as shown in Fig. 3a, but it can also be simply in the form of a number that gives the respiratory rate per unit time.

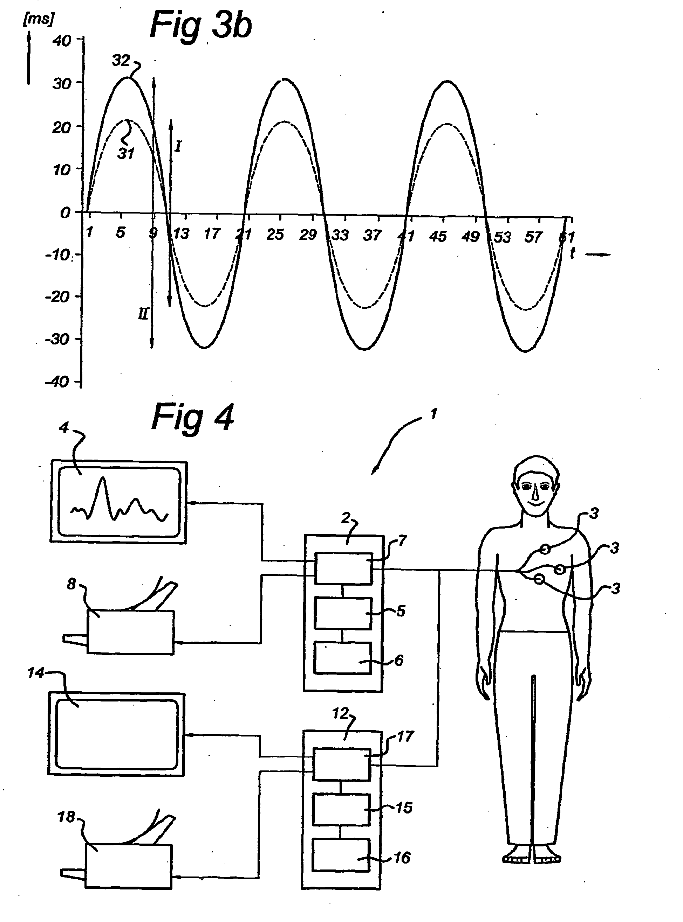

It will be realized that the embodiments discussed here can also be in the form of a separate analytical device 12 as shown in Fig. 4. The analytical device 12 can be connected to the ECG machine 1 either in parallel or in series, as shown in Fig. 4. In the example shown in Fig. 4, the analytical device 12 uses the same electrodes 3 as the ECG machine 1. However, it will be realized that the analytical device 12 can also be connected to the ECG machine 1 in series.

The analytical device 12 can be a computer that comprises a processor 15, a memory 16 and an input-output unit 17; the memory 16 can contain, amongst other things, program instructions that the processor 15 can read and carry out and which enable the processor 15 both to receive data from the electrodes 3 via the input-output unit 17 and to convert these into data that can be output to a suitable output medium, such as a screen 14 or a printer 18 via the input-output unit 17.

The analytical device 12 can of course also function separately, without the ECG machine 1. In this case, the analytical device 12 will comprise a suitable number of electrodes 3.

<u>Flow chart 1</u>

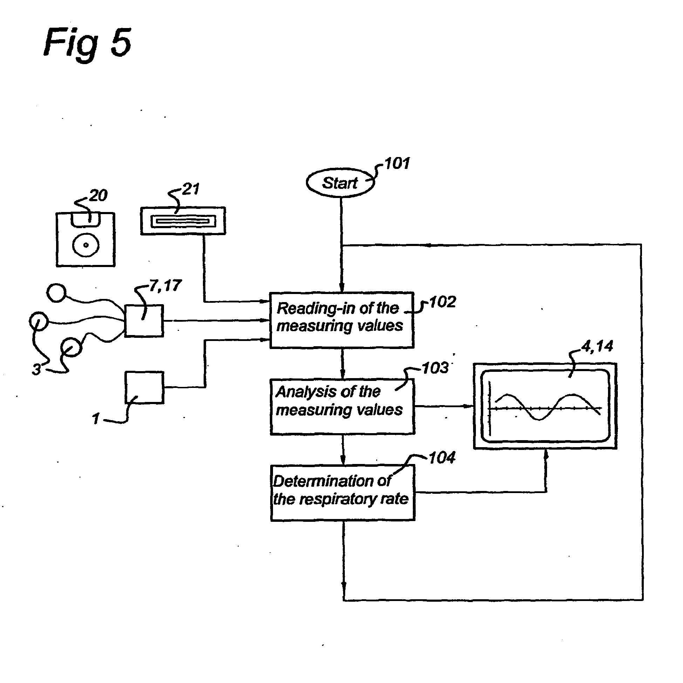

The analytical device 12, which is for example integrated with the ECG machine 1, can contain program instructions that enable the analytical device 12 to carry out the following actions, which are schematically represented in Fig. 5.

The analysis can be started up in a first action 101.

In a second action 102, the processor 5,15 can receive measuring data from the analytical device 12, which are representative of the electrical activity of the heart. These measuring data can be read in by an input-output unit 7, 17 and can come directly from electrodes 3 placed on the body. It should be realized, however, that the measuring data can also come from another device, such as for example a separate ECG machine 1. This is shown in Fig. 5 as an alternative.

Alternatively, the measuring data can be read in from the memory 6,16. The measuring data can also be read in by the analytical device 12 from an information carrier, such as a permanent storage medium, for example a floppy disk, CD-ROM, DVD, USB stick. For this purpose, the analytical device 12 can comprise a suitable reading unit, such as a disk drive or a USB port (not shown). Fig. 5 shows a floppy disk 20 with a disc drive 21, as an alternative.

In a third action 103, the processor 5, 15 can analyse the measuring data and derive from them the successive heartbeats (e.g. by establishing the R peaks) and determine the corresponding RR periods $t_{i-j}$ for performing subsequent processing actions and/or calculations. These can be output by an input-output unit 7,17 to a suitable output medium, such as a screen 4, 14 or a printer 8,18. Suitable interpolation techniques can be used here.

In a fourth action 104, the processor 5, 15 can use the calculated RR periods $t_{i-j}$ to determine the respiratory rate, for example the number of breaths per minute. This value can also be output by the input-output unit 7,17 to a suitable output medium, such as a screen 4, 14 or a printer 8,18.

After completing this fourth action 104, the analytical device 12 can return to action 102. It will be realized that variations in the flow chart illustrated here are possible.

<u>Embodiment 2</u>

In another embodiment, further information is obtained from the electrocardiogram about the respiration of a patient. This can be done by taking a series of values of the RR periods $t_{i-j}$ which collectively represent a respiratory cycle, and by establishing a maximum value $t_{max}$ and a minimum value $t_{min}$ of the RR periods $t_{i-j}$; $t_{max}$ and $t_{min}$ are represented schematically by the curve in Fig. 3a.

It will be realized that $t_{max}$ and $t_{min}$ of the RR periods $t_{i-j}$ need not exactly coincide with actual measuring values of the RR series $t_{i-j}$ in the way shown in Fig. 3a.

As already indicated above, the curve can be obtained, as shown in Fig. 3a by way of example, by means of interpolation, and the crests and troughs then need not necessarily coincide with actual measuring values of the RR periods $t_{i-j}$.

More than one period of the curve shown in Fig. 3a can be reckoned with here. The curve can be determined relatively accurately by applying interpolation to the measuring values $t_{i-j}$ over a relatively long period.

By comparing the maximum value $t_{max}$ and the minimum value $t_{min}$ of the RR periods $t_{i-j}$ with each other,

a measure B of the amount of effort put into the act of taking a breath can be calculated. The measure B of the respiratory effort can be calculated as follows;

$$B = t_{max} - t_{min}.$$

A high value of B indicates that a relatively large effort is made for breathing and can thus indicate breathlessness or stress. A low value of B indicates that a relatively small effort is made for breathing.

To make things clearer, Fig. 3b shows two curves obtained by plotting the successively measured values of the RR period $t_{i-j}$ against time $t_j$. This gives two sinusoidal curves with different amplitudes. Unlike in Fig. 3a, the vertical axis now shows a variation with respect to a mean.

A first curve 31 varies between about +20 ms and -20 ms (with an amplitude marked I), and the curve corresponds to a normal respiratory effort as an example. A second curve 32 varies between about +30 ms and -30 ms (with an amplitude marked II) and corresponds to a raised respiratory effort, for example of someone who is short of breath, given here as an example.

It will be realized that the changes in the respiratory rate can displace the curves shown in Fig. 3b to the left or right along the horizontal axis.

In other words, the amplitude of the curves shown in Figs. 3a and 3b is used to calculate the measure B. When $t_{min}$ falls and/or $t_{max}$ increases, the amplitude becomes greater and so the value of B also increases.

Flow chart 2

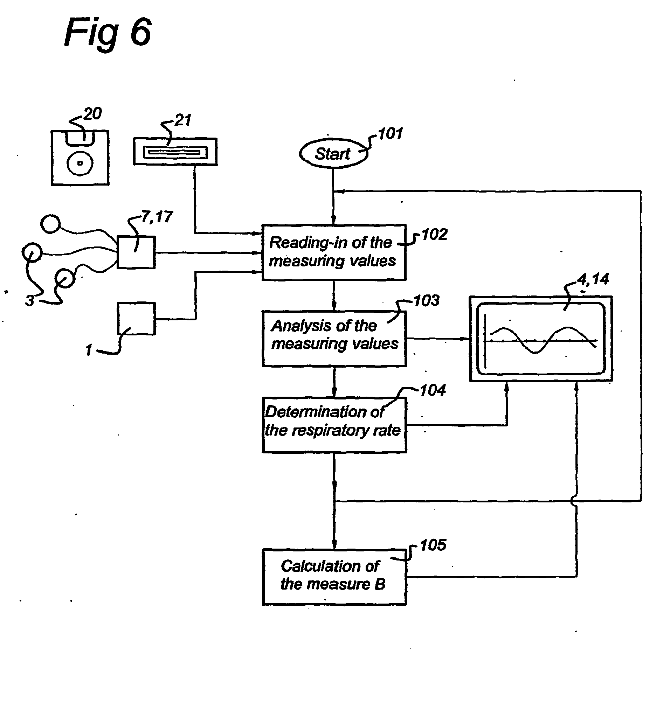

The analytical device 12, which is for example integrated with the ECG machine 1, can comprise program instructions that enable the analytical device 12 to perform this embodiment. These actions are shown schematically in Fig. 6, where actions 101-104 are the same as the actions in Fig. 5.

In this embodiment, the analytical device 12 can also be arranged for calculating the measure B, in a action 105, from the data output in action 103, B being a measure of the amount of effort involved in breathing. This can be done by determining the maximum value $t_{max}$ and the minimum value $t_{min}$ from the data output in action 103 and by calculating the measure B:

$$B = t_{max} - t_{min}.$$

The calculated measure B can be output by the processor 5, 15 via the input-output device 7,17 to a suitable output medium, such as a screen 4, 14 or a printer 8,18.

Embodiment 3

To determine the value of B, it can be compared with a reference measure $B_{ref}$. This reference measure $B_{ref}$ can be obtained in a number of ways.

In a first example, the reference measure $B_{ref}$ can be obtained by carrying out a measurement on the same person or animal before or after. For example, a first measure B1 can be obtained before a certain medication is administered, and a second measure B2 can be obtained after a certain medication is administered. The first measure B1 can then be used as the reference measure $B_{ref}$. If the second measure B2 is lower than the reference measure $B_{ref}$, it can be concluded that the patient needs less effort to breathe. If the second measure B2 is higher than the reference measure $B_{ref}$, it can be concluded that the patient needs more effort to breathe.

In a second example, the reference measure $B_{ref}$ can be obtained from measurements on other, comparable people or animals or groups of people or animals. For example, a measure B is determined for a man aged 65 years, presenting with a certain disorder, and this measure B can be compared with a previously known reference measure $B_{ref}$ that was established earlier for men aged about 65 years and having a comparable disorder.

By comparing the measure B with the reference measure $B_{ref}$ an indicator I can be calculated that indicates whether somebody needs (much) more or less effort to breathe or the usual effort to breathe, where

$$I = B - B_{ref}.$$

A positive value of I indicates that someone needs a well above-average effort to breathe, and a negative value of I indicates that someone needs a below-average effort to breathe. The greater the (positive or negative) value of I, the greater the deviation in the effort needed with respect to the reference measure.

It will be realized that other methods can also be used to calculate I, such as for example:

$$I = (B - B_{ref})/B_{ref}.$$

Flow chart 3

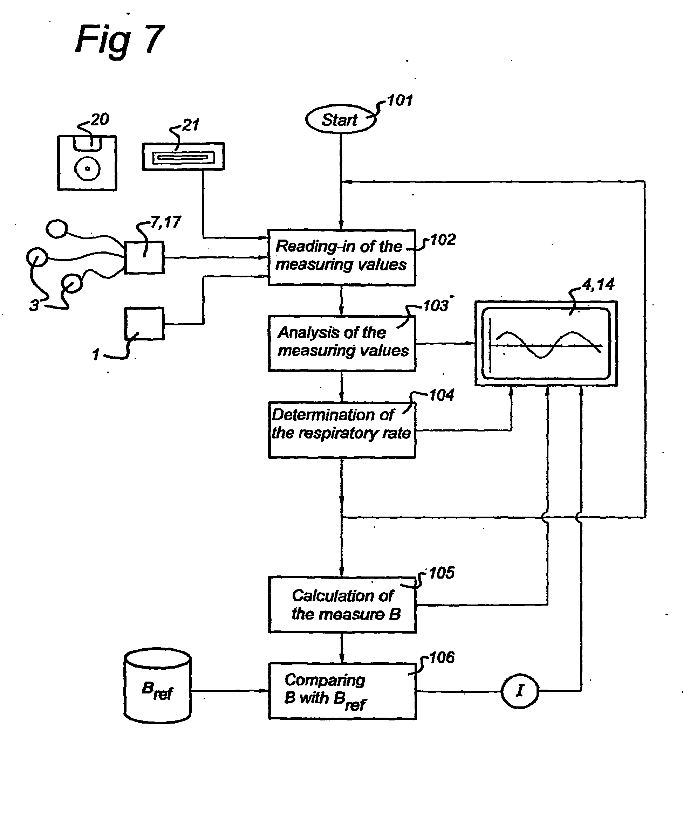

The analytical device 12, which may for example be integrated with the ECG machine 1, can comprise program instructions that enable the analytical device 12 to perform this embodiment. These actions are shown schematically in Fig. 7, where actions 101-105 are the same as the actions in Fig. 6.

In this embodiment, the analytical device 12 can also be arranged for comparing the measure B calculated in action 105 with a reference measure $B_{ref}$ in action 106. The reference measure $B_{ref}$ can be read out of a memory and can be obtained by one of the methods described above.

The comparison of the calculated measure B with the reference measure $B_{ref}$ can be carried out by calculating the indicator I. The processor 5,15 can output the calculated indicator I, via the input-output unit 7,17, to a suitable output medium, such as a screen 4,14 or a printer 8,18.

Advantages

Using the embodiments described above, it is possible to obtain information about the breathing of a human being or an animal in a simple way with the aid of a number of electrodes 3 placed on the patient. Firstly, it is possible to adapt an existing ECG machine 1 to make it suitable for the analysis of a patient's respiration. Secondly, it is also possible to provide a separate analytical device 12 with which information about respiration can be obtained. Such a separate analytical device 12 can easily cooperate with an existing ECG machine 1 by using the same electrodes as the ECG machine 1. Such a separate analytical device 12 can also operate as an independent unit.

Existing ECG machines 1 can easily be adapted to the embodiments described here. Existing ECG machines 1 can be adapted for example by the addition of program instructions that enable the ECG machine 1 to carry out the respiratory analysis.

Existing ECG machines 1 can also be adapted to the embodiments by raising the sampling rate from e.g. 20 ms to 10 ms, 5 ms, 1 ms or even a higher sampling rate in order to be able to determine variations in the RR periods in a sufficiently accurate manner.

According to the embodiments described here, it is possible to use a single ECG curve with a sampling rate higher than the current standard to represent a measure of the respiratory effort (shortness of breath or dyspnoea) per breath in real time, so that information can be obtained amongst other things about (a) the respiratory rate, (b) the respiratory work, (c) fitness, and/or (d) dyspnoea, of a person or animal.

According to the embodiments described above, a patient need not cooperate actively with the respiratory analysis. Thus, the respiration can now be analysed in a simple way in the case of a human being or an animal who or which is not capable of cooperating with a traditional respiratory analysis, such as babies, sleeping subjects, unconscious patients, and animals.

Furthermore, it is sufficient to place a number of electrodes 3 on the person or animal, i.e. no additional sensors are needed, as they are in the case of other, known methods of respiratory analysis which use variations in the pulse transit time (PTT). These methods measure the time needed for a pressure wave generated by a heartbeat to travel to a certain part of the body, for example a finger. This time is called the pulse transit time or PTT.

The velocity of propagation of a pressure wave and so the value of PTT depend on the pressure variations in the chest and on many other factors. The pressure in the chest varies during a respiratory cycle as a result of the movement of the lungs and the thorax. The PTT value therefore also varies with the respiration. To measure the variations in the value of PTT, it is necessary to place electrodes on a patient for measuring the heartbeats, and additionally another sensor must be placed for example on the patient's finger in order to detect the arrival of the pressure wave.

Other comments

As discussed above, the present invention relates to a method and an analytical device for the analysis of respiration. The embodiments described here can be used on a human being or animal at rest (for example during sleep or in a coma), but also during exercise and activities.

As discussed above, an analytical device is provided for the analysis of respiration, which analytical device comprises a processor and an input-output unit, where the analytical device is arranged for

- the reception, via an input-output unit, of measuring data that are representative of the electrical activity of the heart,
- the determination, from the measuring data, of a number of periods of time between successive heartbeats. It has been found that the number of periods of time found to elapse between successive heartbeats can be very useful for obtaining information about a person or animal, in particular information about various parameters concerning the respiration of the said person or animal. The parameters can be derived from the amplitude of the ECG signal, as discussed below, possibly in combination with the measured distances between successive R peaks.

The analytical device can receive measuring data from one of the following: an ECG machine (1), a floppy disk, CD-ROM, DVD, USB stick, but it will be realized that any other suitable medium can also be used, that is to say, any other medium that the analytical device can read.

According to one embodiment, the method further comprises outputting one of a number of periods of time between successive heartbeats, a respiratory rate, measure and indicator.

As discussed above, the method according to one embodiment comprises:

- the determination of the respiratory rate from the number of periods of time found to elapse between successive heartbeats. However, this can also be done per breath.

Mention is made in the application of a relatively high sampling rate of for example 1 ms or less, e.g. 0.1 ms. With this higher sampling rate, the time between 2 successive heartbeats can be determined sufficiently accurately, and the averaging calculation over several respiratory cycles becomes unnecessary. With this sampling rate, the information about breathing and all its derivatives can be determined per respiratory cycle with sufficient accuracy.

However, when at least one respiratory cycle has already been analysed, it is possible to obtain information on the basis of a single determined amplitude of the R peak by comparing it with the information from the previous cycle or previous cycles.

Embodiment 4.

According to another embodiment, a control analysis is proposed, which can be used by the analytical device 12 to check or improve the accuracy of the analysis of respiration on the basis of the variation in a number of periods of time $t_{i-j}$ found to elapse between successive heartbeats, as well as to replace the analysis when the variation in a number of periods of time $t_{i-j}$ found to elapse between successive heartbeats cannot be determined sufficiently accurately. It can happen for example that the RR time variation cannot be determined properly.

The control analysis can take place continuously and in real time.

When for example the RR time variation cannot be determined sufficiently correctly for some reason, it is possible to switch over automatically to the control analysis, and the RR time variation can be "calibrated" against the control analysis.

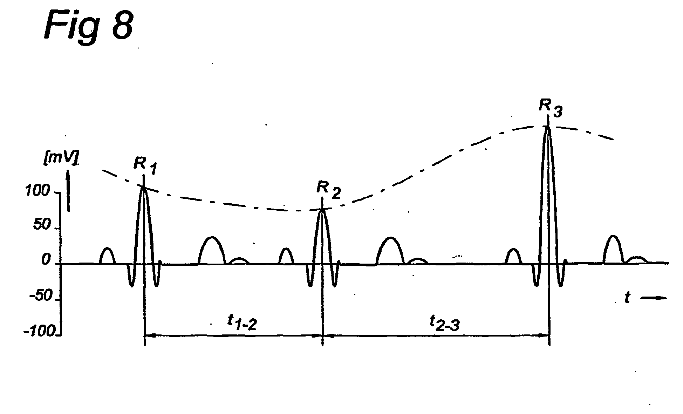

The control analysis will be described below with reference to Fig. 8. Fig. 8 shows schematically an example of an electrocardiogram of the type that can be displayed on a screen 4. Fig. 8 is mostly the same as Fig. 2, but it is clearly shown in Fig. 8 that the amplitude of the R peaks can have different heights. It should be mentioned that the differences in amplitude of the R peaks are somewhat exaggerated in Fig. 8 for the sake of clarity. The control analysis makes use of the fact that the variation in the amplitude of the R peaks follows a sinusoidal course, where the change in amplitude corresponds to the inhalation/exhalation cycle of a human being or an animal. Inhalation corresponds to a small value of both the RR time and the amplitude, while exhalation corresponds to large value of the RR time and the amplitude.

The analytical device 12 can therefore use this variation as a control for the RR time variation. This ensures a refinement of the determination of the RR time variation and all the parameters derived from it. The control analysis can check the calculation based on the RR time variation, make it more accurate or replace it. In this way, it is possible to obtain more accurate information about respiration. It is also possible to obtain information about the respiration under virtually any conditions.

The control analysis also makes use of the relatively high sampling rate and the accurate determination of all the data points in order to be able to determine the exact R peak per heartbeat continuously. This makes it possible to register relatively small variations in the amplitude and to use them in the calculation.

Thus, an analytical device 12 as described above is also provided here, the analytical device 12 being additionally arranged for performing a control analysis in order to check, replace or supplement the periods of time $(t_{i-j})$ between successive heartbeats, which periods of time are determined from the measuring data.

Such an analytical device 12 is furthermore provided in which the control analysis determines the amplitude of a number of R peaks, and also determines the respiratory rate from the difference in amplitude between the number of R. peaks.

Furthermore, a method as described above is provided, which also comprises the performance of a control analysis for checking, replacing or supplementing the number of periods of time $(t_{i-j})$ between successive heartbeats, determined from the measuring data.

Such a method is also provided where the control analysis comprises:

- the determination of the amplitudes of a number of R peaks and
- the determination of the respiratory rate from the difference in amplitude between the number of R peaks.

However, it will be obvious that the control analysis discussed in this embodiment can also be used as an independent means for determining information about respiration, without using the determination of the RR time and its variations.

It will be obvious that the embodiments described above are only given by way of example without any limiting significance, and various modifications and adjustments are possible without leaving the scope of the invention.

**Claims**

1. Analytical device (12) for the analysis of respiration, which analytical device (12) comprises a processor (5, 15) and an input-output unit (7, 17), the analytical device (12) being arranged for

   - the reception, via an input-output unit (7, 17), of measuring data that are representative of the electrical activity of a heart,
   - the determination, from the measuring data, of a number of periods of time ($t_{i-j}$) between successive heartbeats,
which analytical device (12) is arranged for
- the determination of a maximum value ($t_{max}$) of the time ($t_{i-j}$) between successive heartbeats from the measuring data,
- the determination of a minimum value ($t_{min}$) of the time ($t_{i-j}$) between successive heartbeats from the measuring data, and
- the calculation of a measure (B) of the respiratory effort, where the measure (B) is proportional to the difference between the maximum value and the minimum value: $B = t_{max} - t_{min.}$, **characterized in that**
the analytical device (12) is further arranged for
- the reading-in of a reference measure ($B_{ref}$) and
- the calculation of an indicator (I) that shows whether someone has a respiratory effort that is much above or below the average, where the indicator (I) is calculated from the reference measure ($B_{ref}$) and the measure (B).

**2.** Analytical device (12) according to claim 1, which analytical device is arranged to receive measuring data at a sampling rate of 1 ms or less, e.g. 0.1 ms.

**3.** Analytical device (12) according to any one of the preceding claims, which analytical device (12) is also arranged for

- the determination of the respiratory rate from the determined number of periods of time ($t_{i-j}$) between successive heartbeats.

**4.** Analytical device (12) according to any one of the preceding claims, which analytical device (12) is also arranged for outputting, via the input-output unit (7, 17), at least one of a number of periods of time between successive heartbeats, a respiratory rate, a measure (B) and an indicator (1) to a suitable output medium such as a screen (4, 14) or a printer (8, 18).

**5.** Analytical device (12) according to any one of the preceding claims, which analytical device (12) is also arranged for carrying out a control analysis for checking, replacing or supplementing the number of periods of time ($t_{i-j}$) between successive heartbeats, determined from the measuring data.

**6.** Analytical device (12) according to Claim 5, wherein the control analysis determines the amplitude of a number of R peaks and also determines the respiratory rate from the difference in amplitude between the number of R peaks.

**7.** Method for the analysis of respiration, which method comprises:

- the reception of measuring data that are representative of the electrical activity of the heart,
- the determination of a number of periods of time ($t_{i-j}$) between successive heartbeats from the measuring data,
which method also comprises:
- the determination of a maximum value ($t_{max}$) of the period of time ($t_{i-j}$) between successive heartbeats from the measuring data,
- the determination of a minimum value ($t_{min}$) of the period of time ($t_{i-j}$) between successive heartbeats from the measuring data, and
- the calculation of a measure (B) of the respiratory effort, where the measure (B) is proportional to a difference between the maximum value and the minimum value: $B = t_{max} - t_{min.}$, **characterized in that**
the method also comprises:
- the reading-in of a reference measure ($B_{ref}$) and
- the calculation of an indicator (I) that shows whether someone has a respiratory effort that is much above or below the average, where the indicator (I) is calculated from the reference measure ($B_{ref}$) and the measure (B).

**8.** Method according to claim 7, wherein the measuring data received have a sampling rate of 1 ms or less, e.g. 0.1 ms.

**9.** Method according to any one of Claims 7 - 8, which method also comprises:

- the determination of a respiratory rate from the determined number of periods of time ($t_{i-j}$) between successive heartbeats.

**10.** Method according to any one of Claims 7 - 9, which method also comprises outputting one of a number of periods of time between successive heartbeats, a respiratory rate, measure (B) and indicator (I).

**11.** Method according to any one of Claims 7-10, which method also comprises the performance of a control analysis for checking, replacing or supplementing the number of periods of time ($t_{i-j}$) between successive heartbeats, determined from the measuring data.

**12.** Method according to Claim 11, wherein the control analysis comprises:

- the determination of the amplitudes of a number of R peaks and
- the determination of the respiratory rate from the difference in amplitude between the number of R peaks.

13. Computer program which, when loaded onto an analytical device, enables the processor of the analytical device to carry out one of the methods according to Claims 7 - 12.

14. Data carrier, comprising a computer program according to Claim 13.

**Patentansprüche**

1. Analysenvorrichtung (12) für die Atmungsanalyse, welche Analysenvorrichtung (12) einen Prozessor (5, 15) und eine Eingabe-/Ausgabe-Einheit (7, 17) umfasst, wobei die Analysenvorrichtung (12) eingerichtet ist für

   - den Empfang von Messdaten, die für die elektrische Aktivität eines Herzens kennzeichnend sind, über eine Eingabe-/Ausgabe-Einheit (7, 17),
   - die Bestimmung einer Anzahl von Zeitintervallen ($t_{i-j}$) zwischen aufeinanderfolgenden Herzschlägen aus den Messdaten,
   welche Analysenvorrichtung (12) eingerichtet ist für
   - die Bestimmung eines Maximalwerts ($t_{max}$) der Zeit ($t_{i-j}$) zwischen aufeinanderfolgenden Herzschlägen aus den Messdaten,
   - die Bestimmung eines Minimalwerts ($t_{min}$) der Zeit ($t_{i-j}$) zwischen aufeinanderfolgenden Herzschlägen aus den Messdaten, und
   - die Berechnung eines Maßes (B) der Atmungsanstrengung, wobei das Maß (B) proportional zur Differenz zwischen dem Maximalwert und dem Minimalwert: $B = t_{max} - t_{min}$ ist, **dadurch gekennzeichnet, dass**
   die Analysenvorrichtung (12) weiter eingerichtet ist für
   - das Einlesen eines Bezugsmaßes ($B_{ref}$) und
   - die Berechnung eines Indikators (I), der anzeigt, ob jemand eine Atmungsanstrengung aufweist, die sehr über oder unter dem Durchschnitt liegt, wobei der Indikator (I) aus dem Bezugsmaß ($B_{ref}$) und dem Maß (B) berechnet ist.

2. Analysenvorrichtung (12) nach Anspruch 1, welche Analysenvorrichtung eingerichtet ist, um Messdaten bei einer Abtastrate von 1 ms oder weniger, z.B. 0,1 ms, zu empfangen.

3. Analysenvorrichtung (12) nach einem der vorangehenden Ansprüche, welche Analysenvorrichtung (12) auch eingerichtet ist für

   - die Bestimmung der Atmungsrate aus der bestimmten Anzahl von Zeitintervallen ($t_{i-j}$) zwischen aufeinanderfolgenden Herzschlägen.

4. Analysenvorrichtung (12) nach einem der vorangehenden Ansprüche, welche Analysenvorrichtung (12) auch eingerichtet ist, um mindestens eines von einer Anzahl von Zeitintervallen zwischen aufeinanderfolgenden Herzschlägen, einer Atmungsrate, einem Maß (B) und einem Indikator (I) über die Eingabe-/Ausgabe-Einheit (7, 17) zu einem geeigneten Ausgabemedium, wie z.B. einem Bildschirm (4, 14) oder einem Drucker (8, 18), auszugeben.

5. Analysenvorrichtung (12) nach einem der vorangehenden Ansprüche, welche Analysenvorrichtung (12) auch eingerichtet ist, um eine Kontrollanalyse auszuführen, um die Anzahl von Zeitintervallen ($t_{i-j}$) zwischen aufeinanderfolgenden Herzschlägen, die aus den Messdaten bestimmt sind, zu überprüfen, zu ersetzen oder zu ergänzen.

6. Analysenvorrichtung (12) nach Anspruch 5, bei der die Kontrollanalyse die Amplitude einer Anzahl von R-Peaks bestimmt und auch die Atmungsrate aus der Amplitudendifferenz zwischen der Anzahl von R-Peaks bestimmt.

7. Verfahren zur Atmungsanalyse, welches Verfahren umfasst:

   - den Empfang von Messdaten, die für die elektrische Aktivität des Herzens kennzeichnend sind,
   - die Bestimmung einer Anzahl von Zeitintervallen ($t_{i-j}$) zwischen aufeinanderfolgenden Herzschlägen aus den Messdaten, welches Verfahren auch umfasst:
   - die Bestimmung eines Maximalwerts ($t_{max}$) des Zeitintervalls ($t_{i-j}$) zwischen aufeinanderfolgenden Herzschlägen aus den Messdaten,
   - die Bestimmung eines Minimalwerts ($t_{min}$) des Zeitintervalls ($t_{i-j}$) zwischen aufeinanderfolgenden Herzschlägen aus den Messdaten, und
   - die Berechnung eines Maßes (B) der Atmungsanstrengung, wobei das Maß (B) proportional zu einer Differenz zwischen dem Maximalwert und dem Minimalwert: $B = t_{max} - t_{min}$ ist, **dadurch gekennzeichnet, dass** das Verfahren auch umfasst:
   - das Einlesen eines Bezugsmaßes ($B_{ref}$) und
   - die Berechnung eines Indikators (I), der anzeigt, ob jemand eine Atmungsanstrengung aufweist, die sehr über oder unter dem Durchschnitt liegt, wobei der Indikator (I) aus dem Bezugsmaß ($B_{ref}$) und dem Maß (B) berechnet wird.

8. Verfahren nach Anspruch 7, bei dem die empfangenen Messdaten eine Abtastrate von 1 ms oder weniger, z.B. 0,1 ms, aufweisen.

9. Verfahren nach einem der Ansprüche 7 - 8, welches Verfahren auch umfasst:

- die Bestimmung einer Atmungsrate aus der bestimmten Anzahl von Zeitintervallen ($t_{i-j}$) zwischen aufeinanderfolgenden Herzschlägen.

10. Verfahren nach einem der Ansprüche 7 - 9, welches Verfahren auch umfasst: Ausgeben eines von einer Anzahl von Zeitintervallen zwischen aufeinanderfolgenden Herzschlägen, einer Atmungsrate, einem Maß (B) und einem Indikator (I).

11. Verfahren nach einem der Ansprüche 7 - 10, welches Verfahren auch die Ausführung einer Kontrollanalyse umfasst, um die Anzahl von Zeitintervallen ($t_{i-j}$) zwischen aufeinanderfolgenden Herzschlägen, die aus den Messdaten bestimmt sind, zu überprüfen, zu ersetzen oder zu ergänzen.

12. Verfahren nach Anspruch 11, bei dem die Kontrollanalyse umfasst:

- die Bestimmung der Amplituden einer Anzahl von R-Peaks und
- die Bestimmung der Atmungsrate aus der Amplitudendifferenz zwischen der Anzahl von R-Peaks.

13. Computerprogramm, das, wenn es auf einer Analysenvorrichtung geladen ist, ermöglicht, dass der Prozessor der Analysenvorrichtung eines der Verfahren nach den Ansprüchen 7 - 12 ausführt.

14. Datenträger, umfassend ein Computerprogramm nach Anspruch 13.

**Revendications**

1. Dispositif d'analyse (12) pour l'analyse de la respiration, le dispositif d'analyse (12) comprenant un processeur (5, 15) et une unité d'entrée-sortie (7, 17), le dispositif d'analyse (12) étant conçu pour

- recevoir, par le biais d'une unité d'entrée-sortie (7, 17), des données de mesure qui sont représentatives de l'activité électrique d'un coeur,
- déterminer, à partir des données de mesure, un nombre de périodes de temps ($t_{i-j}$) entre des battements cardiaques successifs,
le dispositif d'analyse (12) étant conçu pour
- déterminer une valeur maximale ($t_{max}$) du temps ($t_{i-j}$) entre des battements cardiaques successifs à partir des données de mesure,
- déterminer une valeur minimale ($t_{min}$) du temps ($t_{i-j}$) entre des battements cardiaques successifs à partir des données de mesure, et
- calculer une mesure (B) de l'effort respiratoire, la mesure (B) étant proportionnelle à la différence entre la valeur maximale et la valeur minimale : $B = t_{max} - t_{min}$, **caractérisé en ce que** le dispositif d'analyse (12) est en outre conçu pour
- mémoriser une mesure de référence ($B_{ref}$) et
- calculer un indicateur (I) qui montre si une personne a un effort respiratoire bien au-dessus ou en dessous de la moyenne, l'indicateur (I) étant calculé à partir de la mesure de référence ($B_{ref}$) et de la mesure (B).

2. Dispositif d'analyse (12) selon la revendication 1, le dispositif d'analyse étant conçu pour recevoir des données de mesure à une cadence d'échantillonnage de 1 ms ou moins, par exemple, de 0,1 ms.

3. Dispositif d'analyse (12) selon l'une quelconque des revendications précédentes, le dispositif d'analyse (12) étant également conçu pour

- déterminer la vitesse respiratoire à partir du nombre déterminé de périodes de temps ($t_{i-j}$) entre des battements cardiaques successifs.

4. Dispositif d'analyse (12) selon l'une quelconque des revendications précédentes, le dispositif d'analyse (12) étant également conçu pour transmettre, par le biais de l'unité d'entrée-sortie (7, 17), au moins un d'un nombre de périodes de temps entre des battements cardiaques successifs, d'une vitesse respiratoire, d'une mesure (B) et d'un indicateur (I) à un support de sortie approprié tel qu'un écran (4, 14) ou une imprimante (8, 18).

5. Dispositif d'analyse (12) selon l'une quelconque des revendications précédentes, le dispositif d'analyse (12) étant également conçu pour effectuer une analyse de contrôle pour vérifier, remplacer ou compléter le nombre de périodes de temps ($t_{i-j}$) entre des battements cardiaques successifs, déterminé à partir des données de mesure.

6. Dispositif d'analyse (12) selon la revendication 5, dans lequel l'analyse de contrôle détermine l'amplitude d'un nombre de crêtes R et détermine également la vitesse respiratoire à partir de la différence d'amplitude entre le nombre de crêtes R.

7. Procédé d'analyse de la respiration, le procédé consistant à :

- recevoir des données de mesure qui sont représentatives de l'activité électrique du coeur,
- déterminer un nombre de périodes de temps ($t_{i-j}$) entre des battements cardiaques successifs à partir des données de mesure,
le procédé consistant également à :

- déterminer une valeur maximale ($t_{max}$) de la période de temps ($t_{i-j}$) entre des battements cardiaques successifs à partir des données de mesure,

- déterminer une valeur minimale ($t_{min}$) de la période de temps ($t_{i-j}$) entre des battements cardiaques successifs à partir des données de mesure, et

- calculer une mesure (B) de l'effort respiratoire, la mesure (B) étant proportionnelle à une différence entre la valeur maximale et la valeur minimale : B = $t_{max}$ - $t_{min}$, **caractérisé en ce que** le procédé consiste également à :

- mémoriser une mesure de référence ($B_{ref}$) et

- calculer un indicateur (I) qui montre si une personne a un effort respiratoire bien au-dessus ou en dessous de la moyenne, l'indicateur (I) étant calculé à partir de la mesure de référence ($B_{ref}$) et de la mesure (B).

**8.** Procédé selon la revendication 7, dans lequel les données de mesure reçues ont une cadence d'échantillonnage de 1 ms ou moins, par exemple, de 0,1 ms.

**9.** Procédé selon l'une quelconque des revendications 7 et 8, le procédé consistant également à :

- déterminer une vitesse respiratoire à partir du nombre déterminé de périodes de temps ($t_{i-j}$) entre des battements cardiaques successifs.

**10.** Procédé selon l'une quelconque des revendications 7 à 9, le procédé consistant également à produire l'un d'un nombre de périodes de temps entre des battements cardiaques successifs, d'une vitesse respiratoire, d'une mesure (B) et d'un indicateur (I).

**11.** Procédé selon l'une quelconque des revendications 7 à 10, le procédé consistant également à réaliser une analyse de contrôle pour vérifier, remplacer ou compléter le nombre de périodes de temps ($t_{i-j}$) entre des battements cardiaques successifs, déterminé à partir des données de mesure.

**12.** Procédé selon la revendication 11, dans lequel l'analyse de contrôle consiste à :

- déterminer les amplitudes d'un nombre de crêtes R et

- déterminer la vitesse respiratoire à partir de la différence d'amplitude entre le nombre de crêtes R.

**13.** Programme informatique qui, lorsqu'il est chargé sur un dispositif d'analyse, permet au processeur du dispositif d'analyse de réaliser un des procédés selon les revendications 7 à 12.

**14.** Support de données, comprenant un programme informatique selon la revendication 13.

## Fig 1

## Fig 2

## Fig 3a

*Fig 3b*

*Fig 4*

# Fig 5

# Fig 6

20

21

Start ⌐101

7,17

Reading-in of the ⌐102
measuring values

3

1

Analysis of the ⌐103
measuring values

4,14

Determination of ⌐104
the respiratory rate

Calculation of ⌐105
the measure B

# Fig 7

- Start (101)
- Reading-in of the measuring values (102)
- Analysis of the measuring values (103)
- Determination of the respiratory rate (104)
- Calculation of the measure B (105)
- Comparing B with $B_{ref}$ (106)
- $B_{ref}$
- I
- 20
- 21
- 7,17
- 3
- 1
- 4,14

# Fig 8

**EP 2 037 800 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20050119711 A